Europäisches Patentamt

European Patent Office

Office européen des brevets

(11) Publication number: **0 047 822**
**B1**

# EUROPEAN PATENT SPECIFICATION

(45) Date of publication of patent specification: **30.01.85**

(21) Application number: **81105186.1**

(22) Date of filing: **04.07.81**

(51) Int. Cl.⁴: **A 61 N 1/36, H 02 J 7/00**

(54) Stimulator system.

(30) Priority: **17.09.80 SE 8006523**

(43) Date of publication of application:
**24.03.82 Bulletin 82/12**

(45) Publication of the grant of the patent:
**30.01.85 Bulletin 85/05**

(84) Designated Contracting States:
**AT BE CH DE FR GB IT LI LU NL SE**

(56) References cited:
**FR-A-1 503 915**
**GB-A- 950 182**
**US-A-3 902 502**
**US-A-3 954 111**
**US-A-4 106 511**

(73) Proprietor: **Landskrona Finans AB**
**box 373**
**S-26123 Landskrona (SE)**

(72) Inventor: **Häkansson, Bo Häkan**
**Astrakanvägen 6**
**S-223 56 Lund (SE)**
Inventor: **Saario, Roy Ali**
**Kämnärsvägen 2:136**
**D-222 45 Lund (SE)**

(74) Representative: **Roth, Ernst Adolf Michael et al**
**GÖTEBORGS PATENTBYRA AB Box 5005**
**S-402 21 Göteborg (SE)**

Courier Press, Leamington Spa, England.

## Description

Technical Field

The present invention relates to a stimulator system, comprising a pulse generating means driven by a rechargeable battery, which is adapted so that it generates pulses, transmissible to an electrode support, for the stimulation of a muscle, nerve or the like, the electrode support being adapted so as to be connected to the rest of the system via two points of contact wherein the aforementioned two points of contact are adapted so that they can be connected alternatively to a charging set for the charging of the battery, means being provided to prevent the transmission of stimulating pulses from the said points of contact to the battery via a lead arranged there between for the transmission of energy from the charging set to the battery.

The system preferably comprises an electrode support of the type which has been described in US patent 4 106 511 and EPC publication 0 012 122. The invention can also be applied, however, to other types of pulse-generating electrode supports.

Background Art

In the above-mentioned patents in actual fact one and the same stimulator system is described, which in practice is based on the the utilization of exchangeable batteries.

It is the object of the present invention to provide such a system suitable for the use of rechargeable batteries in accordance with what is customary in other areas where batteries are used for the driving of different apparatuses such as calculators or the like. The invention takes into consideration, however, the special problems which are associated with electrical stimulation, and more particularly, with vaginal or anal stimulation.

US Patent 3 902 502 describes a stimulating system using rechargeable batteries. An essential drawback of this system is, however, the fact that it is not suitable for the use of bipolar pulses and furthermore it needs the use of a high battery voltage.

Disclosure of the Invention

The stimulator system in accordance with the present invention is characterized in that said pulse generating means includes means for generating bipolar pulses of a higher voltage than the voltage of the rechargeable battery and in that said means preventing the transmission of stimulating pulses includes a resistor, a capacitor and a transistor and is arranged in the said transmission lead such that the voltage-dependent element remains non-conductive during pulse generation.

Thanks to this arrangement the bipolar pulses are prevented from being returned to the low voltage battery via the lead intended exclusively for the transmission of energy from the charging set to the battery.

Preferably the system in accordance with the invention, apart from the electrode support and the charging unit, is enclosed in a tight housing with a single outlet, which is common for the electrode support and the charging set.

Between the battery and the voltage-dependent device a rectifier is appropriately connected to prevent any direct effect upon this element or the electrode support from the battery.

Through the invention considerable advantages are achieved, in particular in connection with vaginal stimulation, where the system may easily be exposed to moisture, e.g. in connection with urine leakage on confinement to bed.

It is a considerable advantage that the system can be arranged so that the charging set can never be cut in at the same time as the stimulator. Thus a patient can never be subjected to the direct effect of the charging set.

Moreover, the system can be provided with a completely tight housing with only one opening, as a result of which there should be no problem in achieving a satisfactory seal. If for this purpose a so-called teleplug with two poles is used, this may be identical for the charging set and the electrode support.

In spite of a very simple arrangement of leads, the system can be adapted so that the current is prevented from flowing directly from the battery to the electrode support, whilst charging current may flow in the opposite direction, if the charging set is cut in instead of the electrode support. At the same time, though, stimulator pulses are prevented from passing along the same path as the charging current. Any leakage in this direction is stopped instead by the aforementioned capacitor.

It should be noted that in stimulating human tissues a bipolar stimulation is preferred, that is to say the current flows for one half-cycle in one direction and in the second in opposite direction. In this manner no electrolysis occurs and hence no effect is produced on electrodes or tissues. This entails an additional difficulty in that positive as well as negative pulses must be prevented from leaking back to the battery. However, this problem too is solved by means of the invention, as will be made evident by the following description.

Brief Description of Drawings

Figure 1 shows a preferred embodiment of the stimulator system in accordance with the invention in the form of a block diagram.

Figure 2 shows a typical pulse train, suitable for the stimulator system in accordance with the invention.

Best Mode of Carrying Out the Invention

In the block diagram shown, numeral 1 refers to an electrode support which may be of the type as described in any one of the above-mentioned patents or patent applications. It does not, therefore, need to be described here in detail. When the system is used for electric stimulation, the electrode support is supplied with pulses from a pulse generator 2 which is turn is driven by a battery 3 via an amplifier 4 (voltage multiplier). The electrode support can be connected alternatively with a charging set 5 to two points of contact 6 and 7. In the block diagram this is indicated by means of the contacts 8 and 9 on the electrode support 1. In practice the electrode support and the charging set respectively are connected appropriately with the help of a so-called teleplug which may be of identical design for the two components. The contacts of the charging set have therefore been given the designations 8′ and 9′.

## Charging

During the charging the current through the amplifier 4 and the pulse generator 2 has to be cut out with the help of the contact breaker 10. This can be done manually or automatically as the charging set 5 is cut in. The latter is thus connected to the points of contact 6 and 7 via its contacts 8′ and 9′. The charging current can then flow through the resistor R1 and after recharging of the capacitor C1 via the transistor T and the diode D1 to the positive pole of the battery. At the same time the negative pole of the battery is connected via the point of contact 7 and the contact 9′ in series with the diode D3 to the corresponding pole of the charging set. At the same time no current will be conducted through the diode D2, since the point of contact 6 is at a higher potential than the negative pole of the battery.

## Stimulation

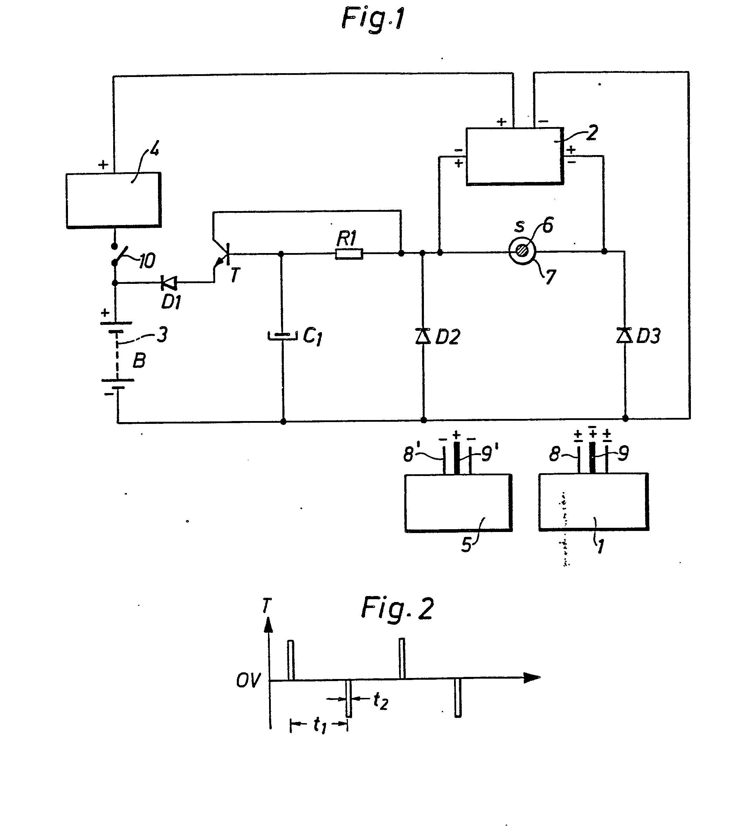

During the stimulation the electrode support 1 is connected to the contacts 6 and 7 instead of the charging set 5. At the same time the contact breaker 10 is closed, so that the battery voltage is transmitted via the amplifier 4 and the pulse generator 2 to the electrode support 1. The positive pulses would then be able to leak back via R1, T and D1. However, this is prevented by choosing the magnitude C1 and R1 in such a manner that a pulse $t_2$ (see Fig. 2) cannot charge the capacitor C1 up to the voltage which is required to make the transistor T conductive. During the time $t_1$, that is to say the time between pulses, the capacitor C1, moreover, can be discharged. The diode D1 is provided because the transistor normally does not withstand such a high back voltage as that which it receives when the negative stimulating pulses arrive.

The diode D2 is provided so that symmetry should be obtained for different directions of current. Otherwise symmetrical pulses are not obtained.

## Example

A suitable stimulator system is obtained when the following electric quantities are chosen for the system.

$t_1 = 50$ ms
$t_2 = 1$ ms
$R_1 = 22$ Ohm
$C_1 = 3.3 \ \mu$F
$D_1 = D_2 = D_3 = 1$N4148 (Siemens)
$T = $ BC 167 (Siemens)
$B = 6$V (5 × 1.2 V NiCd 70mAh) (Gould)

Naturally, though, these values can be varied within wide limits without exceeding the scope of the invention. Similarly, of course, other makes can be used.

For information it may be added that if a chargeable unit is to be produced, the aim is towards reducing the number of chargeable batteries (cells). This is, on the one hand, for reasons of economy, on the other hand for reasons of space. It is also a fact that the energy content per volume diminishes with decreasing cell size.

For a vaginal stimulator of the type which is described in the abovementioned patents and patent applications an output voltage in the order of magnitude of 15V is required. Without voltage amplifier a large number of cells would be required for this. This number can be reduced, however, thanks to the voltage amplifier 4, which is particularly important, if the stimulator is to be portable. It would be possible to bring about the application of bipolar pulses with the help of two voltage units with two sets of cells and doubler. In practice it is simplest, though, by means of the use of two transistors, which alternately supply pulses via two diodes to the electrode support.

The actual generation of pulses can be achieved in a number of different manners, however, and does not constitute a direct part of the invention and need not, therefore, be described in detail here. The essential point is only that in accordance with the invention a pulse generator must be provided, and that the pulses generated by the same must be prevented from getting back to the battery 3 via $R_1$, T and $D_1$.

## Claims

1. A stimulator system comprising pulse generating means (2—4) including a rechargeable battery (3), which is adapted so that it generates pulses, transmissible to an electrode support (1) for the stimulation of a muscle, nerve or the like, the electrode support (1) being adapted so as to be connected to the rest of the system via two points of contact (6, 7), wherein the aforementioned two points of contact (6, 7) are adapted so that they can be connected alternatively to a charging set (5) for the charging of the battery (3), means ($R_1$, $C_1$, T) being provided to prevent the transmission of stimulating pulses from the said points of contact (6, 7) to the battery (3) via a lead arranged there between for the transmission of energy from the charging set (5) to the battery (3), characterized in that said pulse generating means (2—4) includes means for generating bipolar pulses of a higher voltage than the voltage of the rechargeable battery (3), and in that said means preventing the transmission of stimulating pulses includes a resistor ($R_1$), a capacitor ($C_1$) and a transistor (T) and is arranged in the said transmission lead such that the transistor (T) remains nonconductive during pulse generation.

2. A stimulator system in accordance with claim 1, characterized in that the system, apart from the electrode support (1) and the charging set (5) is enclosed in a tight housing with a single outlet which is common for the electrode support and the charging set.

3. A stimulator system in accordance with claim 1 or 2, characterized by a rectifier ($D_1$) connected between the battery and the transistor (T) to prevent any direct effect upon the transistor (T) or the electrode support (1) from the battery (3).

**Revendications**

1. Dispositif de stimulation qui comprend des moyens (2—4) de production d'impulsions, comportant une pile rechargeable (3), qui sont conçus de manière à engendrer des impulsions, transmissibles à un support d'électrode (1) pour la stimulation d'un muscle, nerf ou analogue, le support d'électrode (1) étant conçu pour être connecté au reste du dispositif par l'intermédiaire de deux points de contact (6, 7), ces deux points de contact (6, 7) étant prévus de façon à pouvoir être connectés alternativement à un groupe chargeur (5) pour charger la pile (3), des moyens (R1, C1, T) étant prévus pour empêcher la transmission des impulsions de stimulation des points de contact (6, 7) à la pile (3) par l'intermèdiaire d'un conducteur placé entre ces composants pour la transmission d'énergie du groupe chargeur (5) à la pile (3), caractérisé en ce que les moyens (2—4) de production d'impulsions comprennent des moyens pour engendrer des impulsions bipolaires de tensions supérieure à la tension de la pile rechargeable (3), et en ce que lesdits moyens qui empêchent la transmission des impulsions de stimulation comprennent une résistance (R1), un condensateur (C1) et un transistor (T) et sont placés sur le conducteur de transmission de sorte que le transistor (T) reste non conducteur pendant l'émission d'impulsions.

2. Dispositif de stimulation suivant la revendication 1, caractérisé en ce que le dispositif, sauf le support d'électrode (1) et le groupe chargeur (5), est enfermé dans un boîtier étanche, comportant une sortie unique qui est commune au support d'électrode et au groupe chargeur.

3. Dispositif de stimulation suivant la revendication 1 ou 2, caractérisé en ce qu'un redresseur (D1) est branché entre la pile et le transistor (T) pour empêcher tout effet direct de la pile (3) sur le transistor (T) ou le support d'électrode (1).

**Patentansprüche**

1. Stimulatorsystem mit einem Impulsgenerator (2—4) mit einer wideraufladbaren Batterie (3), die so ausgebildet ist, daß sie auf einen Elektrodenträger (1) übertragbare Impulse für die Stimulierung eines Muskels, Nerves oder dergleichen überträgt, wobei der Elektrodenträger (1) so ausgebildet ist, daß er mit dem Rest des Systems über zwei Kontaktpunkte (6, 7) verbindbar ist, wobei die oben erwähnten beiden Kontaktpunkte (6, 7) derart sind, daß sie alternativ mit einem Ladeaggregat (5) für das Laden der Batterie (3) verbunden werden können, und wobei Einrichtungen ($R_1$, $C_1$, T) vorgesehen sind, um die Übertragung stimulierender Impulse von diesen Kontaktpunkten (6, 7) zu der Batterie (3) über eine für die Übertragung von Energie von dem Ladeaggregat (5) zu der Batterie (3) dazwischen angeordnete Leitung zu verhindern, dadurch gekennzeichnet, daß der Impulsgenerator (2—4) Einrichtungen zur Erzeugung bipolar Impulse einer höheren Spannung als die Spannung der wiederaufladbaren Batterie (3) enthält und daß die Einrichtung zur Verhinderung der Übertragung von stimulierenden Impulsen einen Widerstand ($R_1$), einen Kondensator ($C_1$ und einen Transistor (T) enthält und in der Übertragungsleitung derart angeordnet ist, daß der Transistor (T) während der Impulserzeugung nicht leitend bleibt.

2. Stimulatorsystem nach Anspruch 1, dadurch gekennzeichnet, daß das System abgesehen von dem Elektrodenträger (1) und dem Ladeaggregat (5) in einem dichten Gehäuse mit einem einzigen Auslaß, der für den Elektrodenträger und das Ladeaggregat gemeinsam ist, eingeschlossen ist.

3. Stimulatorsystem nach Anspruch 1 oder 2, gekennzeichnet durch einen Gleichrichter ($D_1$), der zwischen der Batterie und dem Transistor (T) eingeschaltet ist, um eine direkte Wirkung von der Batterie (3) auf den Transistor (T) oder den Elektrodenträger (1) zu verhindern.

0 047 822

Fig.1

Fig.2